(19) Europäisches Patentamt
European Patent Office
Office européen des brevets

(11) Publication number : **0 089 749 B2**

# (12) NEW EUROPEAN PATENT SPECIFICATION

(45) Date of publication of the new patent specification : **10.11.93 Bulletin 93/45**

(51) Int. Cl.[5] : **A61K 7/06,** A61K 7/13

(21) Application number : **83300888.1**

(22) Date of filing : **21.02.83**

## (54) Composition and process for the dyeing of hair.

The file contains technical information submitted after the application was filed and not included in this specification

(30) Priority : **22.02.82 US 350894**

(43) Date of publication of application : **28.09.83 Bulletin 83/39**

(45) Publication of the grant of the patent : **08.11.89 Bulletin 89/45**

(45) Mention of the opposition decision : **10.11.93 Bulletin 93/45**

(84) Designated Contracting States : **AT BE CH DE FR GB IT LI LU NL SE**

(56) References cited :
**DE-A- 2 109 081**
**DE-A- 2 838 878**
**DE-A- 3 024 578**
**FR-A- 2 080 759**
**FR-A- 2 190 406**

(56) References cited :
**GB-A- 1 394 353**
**GB-A- 2 066 663**
**GB-A- 2 068 031**
**US-A- 3 313 734**
**US-A- 3 996 146**
**US-A- 4 273 760**

(73) Proprietor : **Bristol-Myers Squibb Company**
**345 Park Avenue**
**New York, N.Y. 10154 (US)**

(72) Inventor : **Pohl, Stanley H.**
**3 Davenport Avenue**
**New Rochelle New York 10805 (US)**
Inventor : **Hnatchenko, Michael**
**2954 Daniel Street**
**Bronx New York (US)**
Inventor : **Moses, Charles**
**75 Fairlawn Ave., No. 3**
**Bridgeport, Conn. (US)**

(74) Representative : **Jones, Alan John et al**
**CARPMAELS & RANSFORD 43 Bloomsbury Square**
**London, WC1A 2RA (GB)**

EP 0 089 749 B2

## Description

This invention relates to a process and a composition for dyeing hair, more particularly to such composition and process which leaves the hair with a lastingly improved body and combability after the dyeing, which improved effect last through several subsequent shampoos.

During the chemical treatment of hair, especially during dyeing, its body, sheen and manageability are affected to a substantial extent. The restoration of these characteristics is generally referred to as the "conditioning" of the hair. Many materials are known for the conditioning of the hair, but these are generally water soluble and are removed from the hair with the first rinse or shampoo following its application.

### Summary of the Invention

According to the present invention a hair dyeing composition is provided which contains one or more hair dyeing intermediates, the improvement comprising from about 0.5% to about 10.0% of a water soluble cationic polymer product, from about 0.5% to about 30% of a water soluble anionic surfactant, an organic solvent in which the reaction product of the cationic polymer product and the anionic surfactant is soluble, and water, the maximum concentration of which immediately before the use of the composition is limited to an amount which will leave the predominant portions of the anionic surfactant and the cationic polymer product in solution, the composition being adapted to dye hair and also durably to condition when further amounts of water are added to the composition in the presence of hair. The process of the present invention involves the application of the foregoing composition to hair and after a predetermined period rinsing the hair with water.

We have found, surprisingly, that certain cationic polymers defined by the claims, which would normally react with anionic surfactants will not react with such anionic ingredients in the presence of an organic solvent and conventional hair dyeing intermediates (as defined below), but upon the dilution of the composition by additional water, an insoluble conditioning complex will be formed on the hair and, even more surprisingly, will become so firmly bonded to the dyed hair that its conditioning effectiveness will remain present on the hair through several subsequent shampoos. The present invention can be equally carried out on dry hair or premoistened hair.

### Summary of the Prior Art

Cationic polymers are known to be used for the conditioning of hair. U.S. patent No. 3,313,734 discloses a clear, concentrated shampoo composition which is said to provide improved set retention, containing certain cationic polymers and certain detergents. It is stated that a precipitate is formed when the product is diluted during use. The shampoo compositions taught by that patent include specific, commercially unavailable cationic polymers, a large excess (max. tenfold) over the polymer of an essential mixture of anionic and polar nonionic or ampholytic detergents. There is no indication that a durable effect is obtained which lasts throughout several shampoos. This is understandable, because one is not likely to develop a set holding and manageability improving shampoo which will result in a lesser use of that shampoo.

Another clear, acid pH shampoo composition is disclosed in U.S. patent No. 3,996,146. A special multidetergent system is employed for solubilizing the cationic polymer, the system comprising a combination of specific anionic detergents and of specific amphoteric detergents. A number of cationic polymers are listed as useful in the compositions of the patent, however, most of the specific polymers listed are not useful as cationic polymer products in accordance with the present invention. This is because, although these cationic polymers may impart conditioning to the hair on a one shot basis, the conditioning effect is not suggested to endure throughout several shampoos, although it is stated that the attached complex survives a following rinse. This is also borne out by a statement in the patent that a cationic/anionic complex is formed upon dilution with water during shampooing.

Patent No. 3,986,825 deals with the coloring of hair rather than with a shampoo. This patent discloses a hair coloring composition comprised of certain cationic polymers. The presence of an amophoteric, cationic, anionic or nonionic surface active agent is also disclosed, together with sufficient water miscible aliphatic hydroxylated solvent to solubilize the ingredients. The polymers are stated to be equally effective when an anionic, cationic, nonionic or ampholytic agent is present, despite the fact that ionic interaction may lead to the formation of an inactive and insoluble catan wax. The resulting conditioning effect is said to be durable to last throughout several successive rinses or shampoos. Our findings contradict these conclusions, because in accordance with the present invention it is essential that (a) an anionic surfactant be used and (b) that anionic-cationic reaction be substantially prevented until it is caused to occur by the dilution of the composition with copious amounts of water. We found that no durable conditioning can be accomplished when a detergent other than an anionic

detergent is employed. Furthermore, if there is a substantial amount of premature reaction allowed to take place between the cationic polymer product and the anionic detergent, then even if some small measure of conditioning may be observed after use, this conditioning will not be of a durable character that lasts through several subsequent shampoos. This is so, because we noted that the principal amount of the reaction between the anionic detergent and the cationic polymer product has to take place in the presence of the hair to achieve durable bonding thereto. Furthermore, this patent No. 3,986,825 discloses the use of some cationic polymers which were found not to function in accordance with the present invention to impart durable conditioning to hair, which lasts through several shampoos. The conclusions with respect to this patent are further supported by its statement that its conditioning action is achieved even if the application of the composition is followed intentionally or unintentionally by a water rinse. In contrast, the employment of the composition of our invention requires the use of a subsequent water rinse, because only in this manner can the copious amounts of water be imparted to the hair, which will result in the precipitation of the predominant amount of the durably conditioning complex.

GB-A-2 068 031 discloses a process for waving or straightening hair which leaves the hair in a conditioned form which conditioning persists through repeated shampooings which comprises treating said hair with an aqueous waving composition containing an effective amount of a reducing agent and at least one of certain cationic polymers, then treating said hair with an aqueous neutralizing composition containing an oxidizing agent and an anionic or amphoteric detergent.

The present invention provides a hair dyeing composition containing one or. more hair dyeing intermediates and further comprising:

(a) from about 0.5% to about 10% of a water-soluble cationic polymer which has at east one positively charged nitrogen or sulfur moiety in each periodically repeating unit;

(b) from about 0.5% to about 30% of a water-soluble anionic or amphoteric surfactant, said components (a) or (b) forming in aqueous media a substantially water-insoluble reaction product;

(c) an organic solvent in an amount that maintains the hair dye composition as a solution prior to dilution during its use, said dilution being with an amount of water that effects precipitation of the water-insoluble reaction product onto the hair; and

(d) water which in maximum amount is insufficient to precipitate the reaction product in the hair dyeing composition and prior to its use,

said hair dyeing composition being adapted, upon application to hair and in the presence of said dilution amount of water to effect said precipitation of the water-insoluble reaction product onto the hair, to dye and also to durably condition the hair.

## Detailed Description of the Invention

All percentages herein are by weight, based on the composition, except as may be specified otherwise.

The reference to a hair dyeing intermediate, as used throughout the specification and the claims, intends to encompass both oxidative dyeing in which a para component - usually at least one substituted or unsubstituted p-phenylene or p-toluenediamine-and at least one coupler are used to form a dye in the presence of an oxidizer or air, and direct dyeing when a nitro-dye is used directly to dye hair. These dye systems are also referred to in the trade as "permanent" and "semi-permanent" dyes. In the lightening of hair, with or without a preceding bleaching, the employment of toner dyes is also intended to be covered by the reference to a "hair dyeing intermediate".

Without intending to be bound to the following theory, it is assumed that the present invention appears to rely on the effect of the water to dilute the organic solvent which prevents the formation of the water insoluble conditioning precipitate in the presence of hair. As the addition of increasing amounts of water dilutes the solvent effect of the organic solvent, the precipitate which is presumably the reaction product between the cationic polymer product and the anionic surfactant, is formed to an increasing extent.

Wheras there is a one time conditioning effect obtained generally by the precipitate formed as a result of reaction between a cationic polymer and an anionic surfactant, we discovered that a durable conditioning effect is obtained (i.e. lasts through several shampoos) if a cationic polymer of a certain type is used. Such cationic polymers are referred to in the specification and the claims as a "cationic polymer product". That term is defined hereinbelow in greater detail.

Since it is desirable that a predominant portion of the conditioning complex be precipitated in the presence of, and onto, the hair, therefore, it is a requirement of our invention that the amount of water in the composition as it is to be applied to the hair be limited so that the predominant portion of the conditioning precipitate be formed on the hair when the composition as applied to the hair is further diluted by the application of copious amounts of water. For example, when an oxidation dye system is employed in accordance with the present

invention, and the oxidizer is added in the form of its aqueous solution prior to applying the dye composition to hair, the total water content of the composition that is employed should be limited to leave the predominant portions of the cationic polymer product and the anionic surfactant in solution for later precipitation by the addition of further amounts of water to the hair.

In most instances the composition of the present invention is applied to dry hair, but in some cases, such as when the application of the composition follows an earlier bleaching step, then it is applied to wet hair. Although some further precipitation of the durable conditioning complex can be expected to take place when the dyeing composition is applied to wet hair, the amount of the water in the wet hair is relatively low and it is expected that the predominant amount of the precipitation will take place at the time when the dyeing composition is rinsed out of the hair with large amounts of water. The amount of water that can be present in the composition before it is applied to hair will depend on the actual concentrations of the cationic polymer product, the anionic surfactant and the organic solvent in the composition and the conditioning effect that is desired to be achieved. All of these factors can be simply determined by routine experimentation by a person having an average skill in the art.

The term "anionic surfactant" as used throughout the specification and the claims includes in its definition amphoteric surface active agents which become anionic under basic or near to basic conditions if the dyeing of hair carried out in a composition having a basic or near to basic pH, i.e. above the $pk_a$ value of the amphoteric surfactant.

The water soluble cationic polymers that are useful in accordance with the present invention have at least one positively charged nitrogen or sulfur moiety in each periodically repeating unit. The functional quality of the water insoluble durable conditioning complex that is formed on, and adheres to, the hair, which will resist the effect of several subsequent shampooings, depends on a large extent on the characteristics of the particular cationic polymer that is employed. Therefore it is conceivable that some of these cationic polymers which have at least one positively charged nitrogen or sulfur moiety in each periodically repeating unit, will not be as effctive as others. This, in fact, was observed in the base of a number of cationic polymers.

A simple test was devised to determine the usefulness of materials for the conditioning of hair. The test and the associated simple instrumentation are described in an article by M. Garcia et al. in *27* Jnal. Soc. Cosmetic Chemists pp. 379-398 (Sept. 1976) entitled "Combability Measurements on Human Hair", which is incorporated herein by reference thereto. The conditioning of hair is defined for purposes of the present invention as increasing the ease by which a comb can be pulled through freshly washed hair. The term "durable conditioning" as is used throughout the specification and the claims means that after the application of the conditioning composition and the employment of a durable conditioning process, hair is easier to comb after at least three subsequent shampooings, than it is to comb when no conditioning was employed, even after a single shampoo. This defines what is meant by "durable" when we refer to "durable conditioning".

Whether hair is effectively conditioned or not can be simply determined by employing the measurement described in the above-mentioned article on combability measurements. A hair swatch is suspended from an Instron Tensile Tester having a load cell B which has a range of 0-2000 g. Other recording tensile testing instruments can also be used. An attachment is proided having a comb stand, a comb, clamps and the like. The exact character of the measuring device is not critical, since always relative values are determined in comparison to untreated hair. For determining the base line control value for a given hair, the hair swatch is soaked in water, then is combed out until detangled. Then the hair is dipped into water several times to cause controlled tangling. The hair is combed out and the force required to move the comb down through the hair swatch at constant speed is recorded. The effect of treatment on the hair swatch is determined by treating the swatch with an amount of treating agent, in the same manner as treatment is intended to be given to hair in actual use. The hair swatch is then relaxed by a 5 minute immersion in water and again the force is measured that is required to pull a comb through the swatch at constant speed. The difference is then calculated and will provide a measure of the conditioning efficacy of the treatment. In the case of the present invention the.treatment is followed by 3 subsequent shampooings and, therefore, the measurement not only provides information about the conditioning efficacy of the treatment, but also about the durability of the effect.

In the case of the present invention in defining whether we can consider hair to have been conditioned, we cannot employ a scale of absolute force values as could be measured in accordance with the aforementioned article. This is because there are many different kinds of hair, all of which have different combability characteristics. Therefore, it is not unusual that one variety of hair requires a force tenfold or more than the force required to comb another variety of hair that is in a stage of identical conditioning. Notwithstanding this fact, conditioners have been found generally, and especially in the case of the present invention, to improve the combability of all given varieties of hair that were tested.

Accordingly, we have assigned a subjective scale of 5 values to define the ease of combing, i.e. the degree of conditioning of hair, with the value 1 representing the combability of hair that was not conditioned at all. The

value 2 represents a slightly perceptible increase in the ease of combing, but we do not consider hair to have been conditioned until it has a value of at least 3. If hair has a value of 3 after 3 shampooings, then we consider it to have been durably conditioned. It should be kept in mind that these five subjective ease-of-combing values are relative ones and are comparable to each other only when used in each case on the same kind of hair.

According to the arbitrary scale of 5 values, the value 1 was assigned to wet, untreated hair and 5 to the best conditioned value. In accordance with the force measurement that is required for combing, as described above, a maximum conditioning at level 5 would require about 1/10 of the force that is required for an untreated swatch of wet hair. If we consider a level 5 as representing a conditioning improvement of approximately 100%, then we can assign to levels 2, 3 and 4 an approximate improvement of 25%, 50% and 75%, respectively.

As used throughout the specification and the claims, the term "cationic polymer product" denotes a cationic polymer which has at least one positively charged nitrogen or sulfur moiety in each periodically repeating unit and which can form a substantially water insoluble complex when contacted with an ionic surfactant in the presence of hair, said complex durably conditioning the hair.

A number of different cationic polymers were tested to determine whether they satisfy the aforementioned requirements of a cationic polymer product. As a result of such testing it was determined that it is entirely unpredictable from the structural characteristics of a polymer whether or not it will perform as a cationic polymer product. For example, while quaternium-40, a dimethyldiallylammonium chloride homopolymer sold by Merck & Co., Inc. under the name Merquat-100 which is known from DE-A-2109081, performed satisfactorily under the criteria for durable conditioning, as described above and is, therefore, a cationic polymer product, but its low molecular weight (5000) homolog is not, although even a lower molecular weight (3000) cationic polymer (polyquaternium-1) performed satisfactorily.

Other examples of cationic polymers which are not cationic polymer products because they did not perform satisfactorily are given by their official CTFA (Cosmetic, Toiletry and Fragrance Association) names, where available, as contained in the CTFA Cosmetic Ingredient Dictionary (1977 edition), followed by their trademark and source in parentheses. These are:

adipic acid/epoxypropyl diethylenetriamine copolymer (sold by Hercules Chemical Co. under the name Delsette 101);

adipic acid/dimethylaminohydroxyproyldiethylenetriamine copolymer (sold by Sandoz, Inc. under the name Cartaretin F-4);

poly [N-(3-dimethylamino)propyl]-N'-[3-(ethyleneoxyethylene dimethylamino)propyl] urea dichloride (sold under the name Mirapol A15 by Miranol Chemical Co., Inc.);

quaternium-23, a quaternary ammonium polymer formed by the reaction of dimethyl sulfate and a copolymer of vinyl pyrrolidone and dimethylaminoethylmethacrylate (sold by GAF Corporation under the name Gafquat 755N);

quaternium-19, a polymer of hydroxyethyl cellulose reacted with epichlorohydrin and then quaternized with trimethylamine (sold by Union Carbide Corp. under the name Polymer JR-400);

a quaternary ammonium derivative of a hydrolyzed collagen protein (sold by Croda, Inc. under the name of Crotein Q);

quaternium-39, a copolymer of acrylamide and beta-methacryloxyethyl trimethyl ammonium methosulfate (sold by Hercules Chemical Co. under the name Reten 205 M);

aminoethylacrylate phosphate/acrylate copolymer (sold by National Starch Co. under the name Catrex); and

quaternium-41 which is a copolymer of dimethyldiallylammonium chloride with acrylamide (sold by Merck & Co., Inc. under the name Merquat-550).

On the other hand, a number of other cationic polymers were found to perform well as ingredients of durable conditioning compositions in accordance with the present invention and they satisfy the definition of a cationic polymer product.

The cationic polymer products that were found so far are:

polyquaternium-1, a polymeric quaternized dimethylbutenylammonium chloride terminated with quaternized triethanolamine groups, sold by Onyx Chemical Co. under the name Onamer M, hereinafter referred to as "Onamer", and said to have the formula:

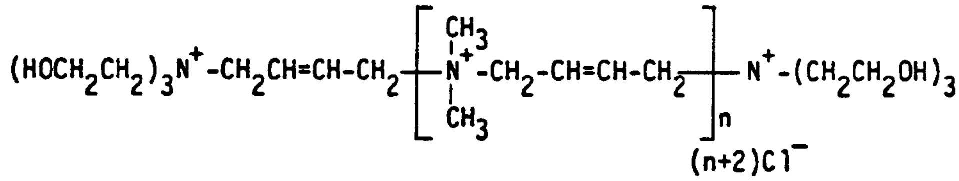

where n is a cardinal number which is proportional to molecular weight;

quaternized poly-4-vinyl pyridine, hereinafter referred to as "QPVP", which is believed to be constituted of repeated units of moiety:

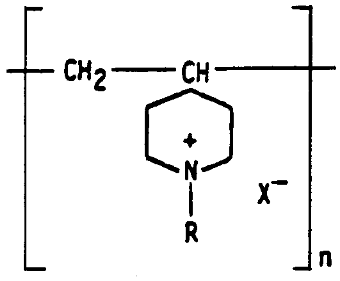

where n is a cardinal number which is proportional to molecular weight, R is a $C_1$-$C_{20}$ alkyl radical and X is a cosmetically acceptable anion such as a halide, sulfate or carboxylate, which can be made by quaternizing and then polymerizing vinylpyridine in a manner known *per se;*

poly (methacrylamidopropyltrimethylammonium chloride), hereinafter referred to as "Clairquat-1", which is made by polymerizing in a manner known *per se* the corresponding monomer sold by Texaco Chemicals under the name MAPTAC and which polymer is said to be constituted of repeating units of the moiety:

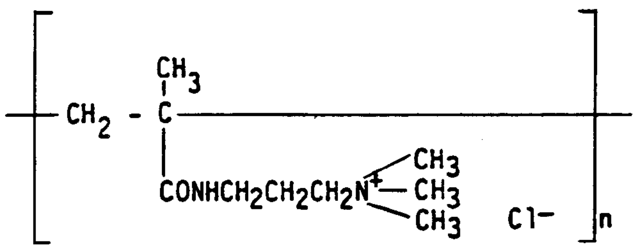

where n is a number which is proportional to molecular weight;

quaternized poly(vinylamine), hereinafter referred to as "QPVAMINE", which can be made by quaternizing and polymerizing vinylamine in a manner known *per se,* and which is believed to be constituted from repeating units of the moiety:

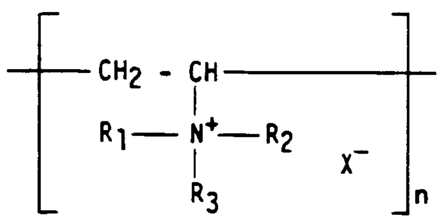

in which $R_1$, $R_2$, and $R_3$ are the same or different and represent $C_1$-$C_{20}$ alkyl groups, and X is a cosmetically acceptable anion such as a halide, sulfate, or carboxylate; and

quaternized poly(ethyleneimine), hereinafter referred to as "QPEMINE", which can be prepared by quaternizing and polymerizing ethyleneimine in a manner known *per se,* and which is believed to be constituted from n repeating units of the monomeric moiety:

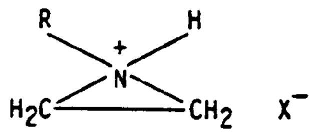

where n is a cardinal number which is proportional to the molecular weight of the polymer, R is a $C_1$-$C_{20}$ alkyl group, and X is a cosmetically acceptable anion such as a halide, sulfate or carboxylate.

In many of the foregoing cationic polymers quaternization can be carried out subsequent to polymerization in a manner known *per se.*

In the foregoing enumeration of specific cationic polymers, whether or not they also are cationic polymer products, the chemical structures are those that were given by the respective manufacturers or were otherwise postulated. Therefore the formulae do not necessarily accurately represent the actual structures of the respective monomeric units that constitute the particular cationic polymer. For example, it was recently learned that the manufacturers of two of the cationic polymer products in the foregoing listing changed their views about the formulae which they initially provided for their respective cationic polymers without changing their respective products. The new structural representations, as provided by their respective manufacturers, appears in this application with respect to those two cationic polymer products. It is for that reason that we do not wish to be bound to any specific structural representation of any cationic polymer product herein, but in each case view the manufacturer's trademark, the CTFA designation and any chemical name (the latter usually based on what was done to a precursor to obtain the end product), to be of equal significance in defining each cationic polymer product.

All of the aforementioned cationic polymer products, which by definition of that term, will work in accordance with the present invention were determined to have a value between 3 and 5 on the combability test described above and conducted after 3 shampooings subsequent to the application of the composition. Thus their ability has been established to impart durable conditioning properties to compositions and to hair treated thereby.

Since it has been demonstrated that the functioning of the cationic polymer in accordance with the durable conditioning features of the present invention cannot be predicted, and since it has also been demonstrated how the suitability of a polymer within the above-identified group can be established by simple routine experimentation, therefore, the term "cationic polymer product", as used throughout the specification and the claims, includes all cationic polymers of the type defined above. Thus the term "cationic polymer product", as used throughout the specification and the claims, includes in its definition not only cationic polymers which have been specifically enumerated herein, but also any and all other cationic polymers which are a "cationic polymer product", as defined hereinabove, as the present invention does not reside in the identity of any given cationic polymer product, nor does it reside only in the employment of any given cationic polymer product, but in the manner that any cationic polymer product is employed.

As it can be readily appreciated, the term "cationic polymer product" is not restricted to homopolymers, but copolymers of multiple monomers are intended to be included in the meaning of the term.

Those specific cationic polymers that were enumerated above as being cationic polymer products, were found to perform, with one exception over a wide range of molecular weights between about 4,000 to about 550,000; most of them suitably from about 20,000 to about 100,000. The only exception that was found so far is Onamer which provides an effective cationic polymer product at a molecular weight between about 1,000 and about 3,000. The manner of determining and expressing the molecular weight makes no difference in this case.

The term "cationic polymer product" as used throughout the specification and the claims also includes mixtures of one or more cationic polymer products. As employed in accordance with the present invention cationic polymer product was found to be sufficient to be present in the composition in concentration between about 0.5% to about 10%, suitably from about 2% to about 5%. The optimum concentration can vary between different cationic polymer products. For example, in the case of Onamer M about 5% is a practical low limit, while in the case of Merquat-100 effective durable conditioning could be obtained at a concentration of about 2.5%.

The mechanism of the durable conditioning reaction obtained in accordance with the present invention is not clearly understood. We believe that initially the positively charged sites of the cationic polymer product component of the composition forms a bond with the negatively charged sites of the hair which are formed during dyeing. Up to this point the assumed mechanism is similar to the known, normal conditioning of hair when positively charged monomeric or polymeric quaternary amines are employed for conditioning. While we postulate that, in accordance with our invention, the remaining free positive charges of the cationic polymer product react in the presence of hair with the anionic surfactant to form the durable conditioning complex on the hair which

remains attached to the hair and conditions it through several shampoos without need for reapplication each time, we do not wish to be bound by this speculative assertion.

It was found, surprisingly, that in most cases durable conditioning could be obtained at most pH levels. Hair is built of amino acids. These are basic molecular units which contain both positive and anionic charges. When the final polymeric character of the hair is established, there is generally believed to be an equal number of the negative and positive charges present in the hair. Thus hair can be termed to be an amphoteric bipolymer. The neutrality of that system, which means that there is a region of pH at which the number of positive charges and the number of negative charges are equal, is called an isoelectric region. That isoelectric region in hair is approximately pH 4. Above pH 4, on the basis of the charges present in the hair, any agent, such as moistening the hair, should render the hair negatively charged. An agent at a pH below 4 should render the hair positively charged. While this is a theorectical dividing line in practice we find that, possibly due to the wearing properties of the hair, the surface characteristics of hair do not mirror our chemical calculations, which means that the head is able to pick up cationic materials at a pH even lower than 4. It could only pick up cationic materials if the hair surface was negatively charged; that probably can extend down to about pH 2. We can measure this activity of the cationic material by treating the hair with radioactively tagged cationic surfactants and measure the radioactivity of hair. When this is done it is found that hair has approximately no charge in terms of its surface activity or surface affinity, at about pH 2. Any cationic polymer product above pH 2 is likely to be attached to hair, although more of the surfactants are expected subsequently to be bound at pH 7 than at pH 4. Furthermore, if an amphoteric surfactant is employed, then it should be used at basic pH at which it becomes anionic.

Although it is theoretically possible to employ our invention at low pH levels, as a practical matter, the dyeing of hair is generally carried out at or above pH 8. The customary hair dyes and dye intermediates and ingredients can be employed along with the new essential ingredients of the compositions of the present invention. Therefore, the hair dye systems do not require further elaboration.

We are not aware of any water soluble anionic or amphoteric surfactants that cannot be successfully employed in accordance with the present invention.

Two classes of amphoteric detergents have been found to be particularly suitable. The first class can be defined by the formula:

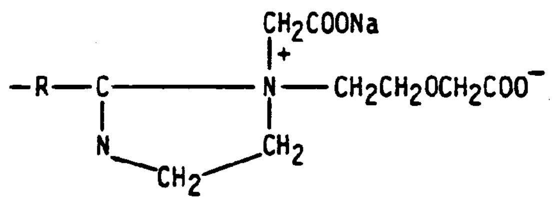

in which R is a long chain fatty radical containing from 10 to 18 carbon atoms. A typical example of such a compound or compounds is the case in which R represents coconut fatty radicals. A material of this character is sold under the trade name MIRANOL C2MSF by the Miranol Chemical Company, Inc., with its CTFA name being amphoteric-2.

A second class of amphoteric detergents that is particularly effective for the purpose of the present invention can be defined by the formula:

$$R\text{-}NH\text{-}CH_2\text{-}CH_2\text{-}COOH$$

wherein R is a long chain fatty alkyl group having from 10 to 18 carbon atoms. An example of such a detergent is marketed under the trade name DERIPHAT 170C by General Mills Chemicals, Inc., with its CTFA name being lauraminopropionic acid in which the R is a mixture of lauryl and myristyl fatty alkyl groups.

All anionic surfactants tested have been found useful in this invention; thus, salts of alkyl sulfonates, alkyl sulfates, sulfonated and sulfated alkyl ethers as well as long chain carboxylic acid (where the chain length is constituted of at least 10 carbon atoms) exhibit formation of durable conditioning complexes. By way of specifically illustrating suitable anionic detergents we mention sodium lauryl sulfate, sodium lauryl ether sulfate, TEA lauryl sulfate, sodium stearate, etc.

The quantity of amphoteric or anionic detergent which will be contained in the composition of this invention will vary somewhat depending on the economics and the results sought. However, usually this will be in the range of from about 0.5% to about 30% and suitably in the range of from about 5% to about 20%.

The solvent which maintains the hair dye composition in solution can be conveniently aliphatic alcohols or ethers, such as ethanol, isopropanol, butanol, carbitol and the like. Polyhydroxy compounds such as glycols, also alkyl phenols, ethoxylated solvents, glyceryl fatty acids, aliphatic phenyl ethers and the like can also be used, because even if some might not be good solvents by themselves, their solvent action can be potentiated

by the addition of a lower boiling point solvent such as an aliphatic alcohol. The presence of any higher boiling point ingredients can be advantageous in reducing the volatility of the solvent.

Some observations indicate that it appears that the durable bonding of the anionic-cationic complex to the hair is promoted by the negative charges that are formed on the hair, resulting in a strong bond with free positive charges of the precipitated complex or the cationic polymer product. This appears to be borne out by the observation that in view of the better results which can be obtained when there is an excess of a cationic polymer product over the anionic surfactant, the concentration of those ingredients may be somewhat lower than in the absence of such an excess. The weight ratio of the cationic polymer product to the anionic detergent can suitably be varied from about 1:2 to about 2:1. As indicated, in the former case a higher concentration of both ingredients may have to be employed than in the latter case.

Our invention is further illustrated by the following examples, but the full scope of the invention is defined by the claims.

## Examples

In the following examples all figures are given as percentages by weight. Chemical names and CTFA names are used throughout.

The formulae of the examples were mixed with an equal weight of a 6% hydrogen peroxide aqueous solution and are then applied to hair for a period of 5-15 minutes.

In the following table a number of ingredients have more than one function. In some cases they are relied on for more than one of these properties. The ingredients A and B are para components, C-E are couplers, F and G are reducing agents used as preservatives, H is a chelating agent to remove any metal ion contaminants, I and J are alkalizers, K-P are solvents for the dye and for purposes of the invention P-S are wetting agent and thickeners, T-V are anionic surfactants and consistency adjusters, W-Y are cationic polymer products, Z and AA are used to neutralize some excess anionic surfactant content.

The compositions of the examples 1-11 are applied to swatches of hair and the qualitative evaluation of the results shows the dyed hair strands to be dyed as well as conditioned by the treatment of the examples.

The examples are presented in a tabular form wherein the ingredients are represented by letters, as explained in the following table:

| | |
|---|---|
| A | p-phenylene diamine |
| B | N,N-bis-hydroxyethyl-p-phenylenediamine sulfate |
| C | resorcinol |
| D | 1-naphthol |
| E | 2-methylresorcinol |
| F | sodium sulfite |
| G | erythorbic acid |
| H | ethylenediaminetetraacetic acid |
| I | ammonia (28%) |
| J | ethanolamine |
| K | ethoxydiglycol |
| L | propylene glycol |
| M | hexylene glycol |
| N | ethanol |
| O | isopropyl alcohol |
| P | linoleic acid diethanolamide |
| Q | octoxynol-1 |
| R | nonoxynol-4 |
| S | nonoxynol-9 |
| T | oleic acid |
| U | sodium lauryl sarcosinate |
| V | sodium lauryl sulfate (30%) |
| W | onamer M (40%) |
| X | QPVP (25% in Examples 1-4 and 11 and 28% in Examples 5-9) |
| Y | clairquat-1 |
| Z | quaternium-18 |
| AA | quaternium-34 |
| BB | N",N"-bis(2-hydroxyethyl)-N-methyl-2-nitro-p-phenylenediamine |
| CC | N-(2-hydroxyethyl)-2-nitroaniline |

In the following examples a horizontal line means that the particular ingredient was not employed in that example.

| | Examples | | | | | | | | |
|---|---|---|---|---|---|---|---|---|---|
| Ingredient | 1 | 2 | 3 | 4 | 5 | 6 | 7 | 8 | 9 |
| A | 0.01 | - | 0.20 | - | 0.20 | - | - | 0.10 | 0.40 |
| B | 0.001 | - | 0.05 | - | 0.095 | - | - | 0.05 | 0.02 |
| C | 0.02 | - | 0.15 | - | 0.60 | - | - | 0.40 | 0.40 |
| D | 0.005 | - | - | - | 0.05 | - | - | 0.05 | 0.01 |
| E | - | - | - | - | 0.10 | - | - | - | - |
| F | 0.10 | 0.20 | 0.10 | 0.10 | 0.10 | 0.20 | 0.20 | 0.20 | 0.20 |
| G | 0.20 | - | 0.20 | - | 0.20 | - | - | - | - |
| H | 0.10 | 0.10 | 0.10 | 0.10 | 0.02 | 0.10 | 0.10 | 0.10 | 0.10 |
| I | - | - | 9.50 | - | 9.50 | - | - | 9.0 | - |
| J | 10.0 | 4.0 | - | 3.50 | - | 3.50 | 3.50 | - | 3.0 |
| K | 5.0 | 5.0 | 6.90 | 5.0 | 6.40 | 5.0 | 5.0 | 4.0 | 5.0 |
| L | 3.0 | 3.0 | 8.80 | 3.0 | 8.80 | 3.0 | 3.0 | 4.0 | 8.8 |
| M | - | 4.0 | - | 4.0 | - | - | - | - | - |
| N | 10.0 | 10.0 | - | 10.0 | - | 10.0 | 10.0 | 11.0 | 10.0 |
| O | - | - | 8.0 | 8.0 | 8.00 | - | - | - | - |
| P | 3.0 | 2.50 | - | 2.50 | - | - | 2.50 | - | - |
| Q | 13.0 | 15.0 | 8.40 | 15.0 | - | - | - | - | - |
| R | - | - | 4.20 | - | 4.20 | - | - | - | - |
| S | 13.0 | 11.0 | - | 11.0 | - | 11.0 | 11.0 | 13.0 | 11.0 |
| T | 6.50 | 5.0 | 21.0 | 5.0 | 21.0 | 5.0 | 5.0 | 5.0 | 5.0 |
| U | - | - | 3.0 | 3.0 | 3.0 | - | - | - | - |
| V | 2.50 | 2.50 | - | 2.50 | - | 2.50 | 2.50 | 2.0 | 2.0 |
| W | 9.0 | 9.0 | 7.5 | - | 7.5 | - | 9.0 | - | - |
| X | - | - | - | 16.0 | - | 12.90 | - | - | 12.0 |
| Y | - | - | - | - | - | - | - | 5.0 | - |
| Z | - | - | - | - | 2.50 | - | - | - | - |
| AA | - | - | 2.50 | 2.50 | - | - | - | - | - |

water to 100% in each case.

Semipermanent Dyes

| Ingredient | Examples 10 |
|---|---|
| BB | 0.2 |
| CC | 0.2 |
| O | 8.0 |
| K | 15.0 |
| R | 5.0 |
| S | 4.0 |
| J | 7.0 |
| T | 2.0 |
| V | 1.0 |
| X | 8.0 |
| Water | 49.6 |

The compositions of some examples were also tested for the durability of the conditioning effect after several shampoos. The wet combing force was measured by the test described earlier in this application. The effect of the conditioning and of subsequent shampoos is shown in the following table, expressed as a change (+ increase, - decrease) in the force required for combing, in comparison to the force required to comb the untreated hair swatch.

| | change in wet combing force | | |
|---|---|---|---|
| Treatment | After dyeing | + 1 Shampoo | +2 Shampoos |
| Standard commercial hair dye | +45% | +253% | +237% |
| Example 5 | +4% | +68% | +70% |
| Example 1 | -67% | -56% | -22% |
| Example 2 | -32% | -11.6% | -3.5% |

The invention is defined by the following claims.

## Claims

1. An aqueous hair dyeing composition containing one or more hair dyeing intermediates and further comprising:
   (a) from about 0.5% to about 10% of a water-soluble cationic polymer which has at least one positively charged nitrogen moiety in each periodically repeating unit; said cationic polymer being from
      (i) polyquaternium-1, a polymeric quaternized dimethylbutenyl-ammonium chloride terminated with quaternized triethanolamine groups, and said to have the formula:

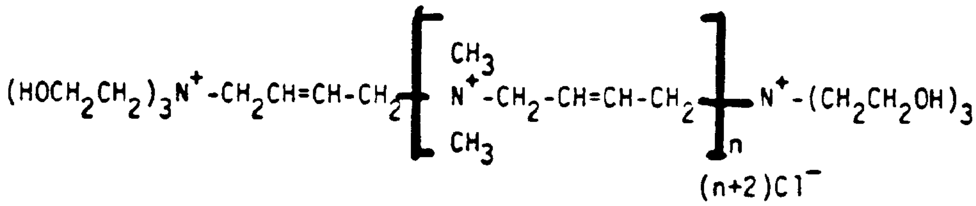

wherein n is a cardinal number which is proportional to molecular weight;

(ii) quaternized poly-4-vinyl pyridine, referred to as "QPVP", which is believed to be constituted of repeated units of moiety:

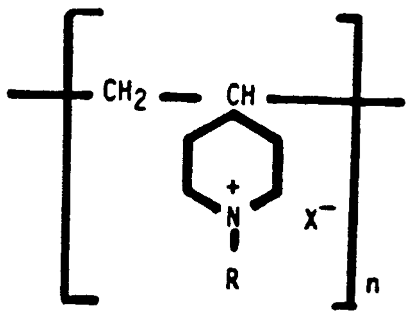

where n is a cardinal number which is proportional to molecular weight, R is a $C_1$-$C_{20}$ alkyl radical and X is a cosmetically acceptable anion such as a halide, sulfate or carboxylate, which can be made by quaternizing and then polymerizing vinylpyridine in a manner known *per se*;

(iii) poly(methacrylamidopropyltrimethylammonium chloride), referred to as "Clairquat-1" (Trade Mark), said to be constituted of repeating units of the moiety:

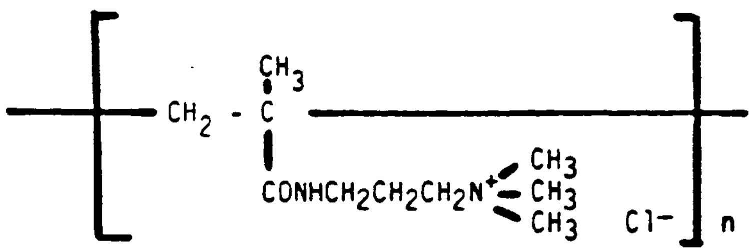

where n is a cardinal number which is proportional to molecular weight;

(iv) quaternized poly(vinylamine), referred to as "QPVAMINE", which is believed to be constituted from repeating units of the moiety:

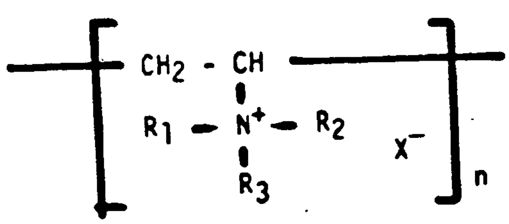

in which $R_1$, $R_2$, and $R_3$ are the same or different and represent $C_1$-$C_{20}$ alkyl groups, and X is a cosmetically acceptable anion such as a halide, sulfate, or carboxylate; and

(v) quaternized poly(ethyleneimine), referred to as "QPEMINE", which is believed to be constituted from n repeating units of the monomeric moiety:

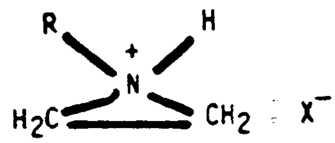

where n is a cardinal number which is proportional to the molecular weight of the polymer, R is a $C_1$-$C_{20}$ alkyl group, and X is a cosmetically anion such as a halide, sulfate or carboxylate;

(b) from about 0.5 to about 30% of a water-soluble anionic or amphoteric surfactant, said components (a) and (b) forming in dilute aqueous media a substantially water-insoluble reaction product;

(c) an organic solvent in an amount that maintains the aqueous hair dye composition as a solution prior to dilution during its use, said dilution being with an amount of water that effects precipitation of the water-insoluble reaction product onto the hair; and

(d) water which in maximum amount is insufficient to precipitate the reaction product in the hair dyeing composition and prior to its use;

said hair dyeing composition having a pH of 8 or more and being adapted, upon application to hair and in the presence of said dilution amount of water to effect said precipitation of the water-insoluble reaction product onto the hair, to dye and also durably to condition the hair.

2. A hair dyeing composition according to claim 1 wherein said hair dyeing intermediate is an oxidation hair dye intermediate, a direct dye or a mixture of an oxidation dye intermediate with a direct dye.

3. A process for dyeing and durably conditioning hair, which comprises applying to hair the dyeing composition according to either of claims 1 and 2, and after a predetermined period rinsing the hair with water, whereby the water-insoluble reaction product is precipitated and is deposited onto the hair.

## Patentansprüche

1. Wässrige Haarfärbezusammensetzung, enthaltend eines oder mehrere Haarfärbezwischenprodukte und zusätzlich:

(a) etwa 0,5 % bis etwa 10 % eines wasserlöslichen kationischen Polymers, welches mindestens einen positiv geladenen Stickstoffrest in jeder sich wiederholenden periodischen Einheit besitzt; wobei das genannte kationische Polymer ausgewählt ist aus

(i) Polyquaternium-1, ein polymeres quaternisiertes Dimethylbutenyl-Ammoniumchlorid, welches terminale quaternisierte Triethanolamingruppen besitzt und welchem die Formel:

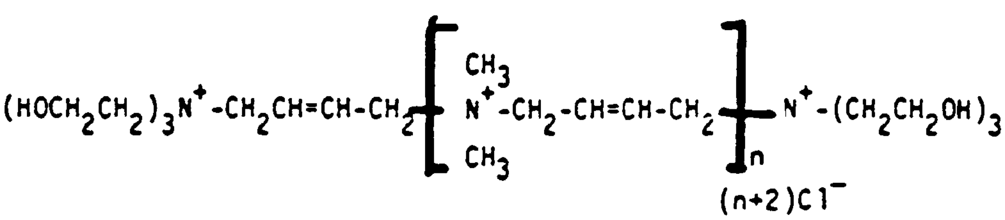

zugeschrieben wird, worin n eine Kardinalzahl ist, die proportional zum Molekulargewicht ist;

(ii) ein quaternisiertes Poly-4-vinylpyridin, welches als "QPVP" bezeichnet wird, von welchem angenommen wird, dass es sich aus den sich wiederholenden Einheiten des Teils:

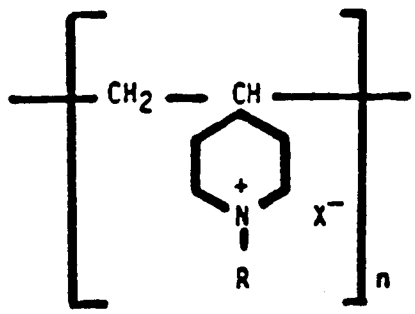

zusammensetzt, worin n eine Kardinalzahl darstellt, welche proportional zum Molekulargewicht ist, R ein $C_1$-$C_{20}$ Alkylrest ist und X ein kosmetisch annehmbares Anion, wie Halogenid, Sulfat oder Carboxylat, ist, welches quaternisiert und dann in bekannter Weise das Vinylpyridin polymerisiert werden kann;

(iii) Poly(methacrylamidopropyltrimethylammoniumchlorid), welches als "Clairquat-1" (Warenzeichen) bezeichnet wird, von welchem angenommen wird, dass es sich aus sich wiederholenden Einheiten des Restes:

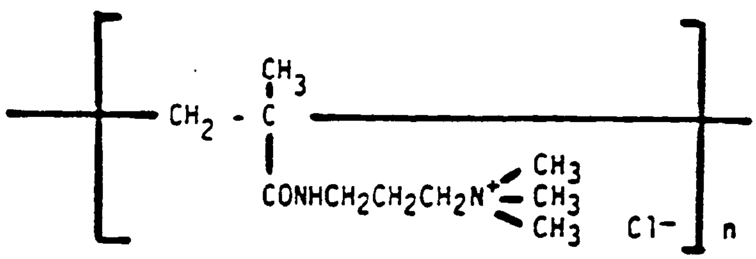

zusammensetzt, worin n eine Kardinalzahl ist, welche zum Molekulargewicht proportional ist;

(iv) quaternisiertes Poly(vinylamin), welches als "QPVAMIN", bezeichnet ist, von welchem angenommen wird, dass es aus sich wiederholenden Einheiten des folgenden Restes zusammensetzt:

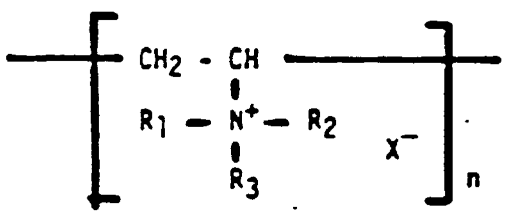

worin $R_1$, $R_2$ und $R_3$ gleich oder verschieden sind und $C_1$-$C_{20}$ Alkylgruppen darstellen und X ein kosmetisch annehmbares Anion, wie Halogenid, Sulfat oder Carboxylat ist; und

(v) quaternisiertes Poly(ethylenimin), welches als "QPEMIN" bezeichnet wird, von welchem angenommen wird, dass es sich aus n sich wiederholenden Einheiten des monomeren Restes:

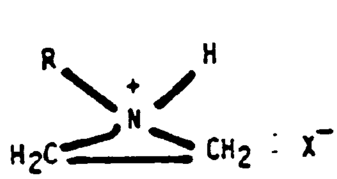

zusammensetzt, worin n eine Kardinalzahl ist, welche zum Molekulargewicht des Polymers proportional ist, R eine $C_1$-$C_{20}$ Alkylgruppe ist und X ein kosmetisches Anion wie ein Halogenid, Sulfat oder Carboxylat ist;

(b) etwa 0,5 bis etwa 30 % eines wasserlöslichen anionischen oder amphoterischen oberflächenaktiven Mittels, wobei die genannten Komponenten (a) und (b) in verdünnten wässrigen Medien ein im wesentlichen wasserunlösliches Reaktionsprodukt bilden;

(c) ein organisches Lösungsmittel in einem Anteil, welcher die wässrige Haarfärbezusammensetzung als Lösung erhält vor seiner Verdünnung während seiner Verwendung, wobei die genannte Verdünnung mit einer Menge Wasser erfolgt, welche eine Ausfällung des wasserunlöslichen Reaktionsproduktes in das Haar erlaubt; und

(d) Wasser in einem maximalen Anteil, welcher ungenügend ist um das Reaktionsprodukt in der Haarfärbezusammensetzung vor der Verwendung auszufällen;

wobei die genannte Haarfärbezusammensetzung einen pH-Wert von 8 oder mehr aufweist und angepasst ist an die Anwendung auf das Haar und an die Gegenwart der genannten zur Verdünnung erforderlichen Wassermenge um die genannte Ausfällung des wasserunlöslichen Reaktionsproduktes auf das Haar zu bewirken, zu färben und das Haar dauerhaft zu konditionieren.

2. Haarfärbezusammensetzung gemäss Anspruch 1, worin das genannte Haarfärbezwischenprodukt ein Oxidationsfärbezwischenprodukt, ein Direktfarbstoff oder eine Mischung eines Oxidationsfärbezwischenproduktes mit einem Direktfarbstoff ist.

3. Verfahren zur Färbung und dauerhaften Konditionierung von Haar, umfassend die Applikation der Färbezusammensetzung gemäss Anspruch 1 oder 2 auf das Haar und Spülung des Haars nach einer vorbestimmten Zeitperiode mit Wasser, wobei das wasserunlösliche Reaktionsprodukt ausgefällt wird und auf dem Haar abgelagert wird.

## Revendications

1. Une composition aqueuse de teinture de cheveu contenant un ou plusieurs intermédiaires de coloration et comprenant en outre:

(a) d'environ 0,5% à environ 10% d'un polymère cationique hydrosoluble comportant, dans chacun de ses motifs répétitifs, au moins un groupe azoté chargé positivement, ledit polymère cationique étant formé de:

(i) polyquaternium-1, un chlorure de diméthylbuténylammonium quaternisé ayant à ses extrémités terminales des groupes triéthanolamine quaternisés et présentant la formule suivante:

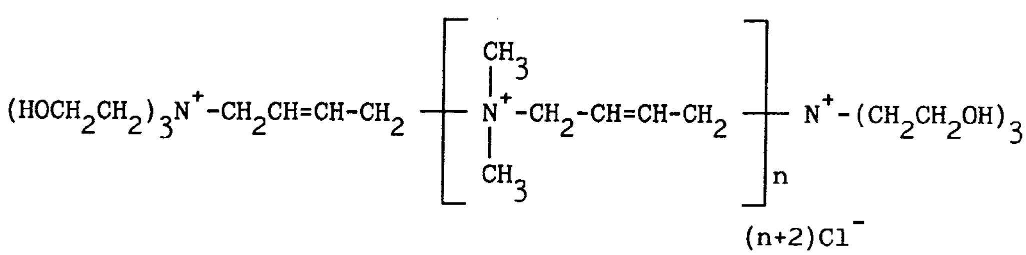

dans laquelle n est un nombre cardinal proportionnel au poids moléculaire;

(ii) poly-4-vinylpyridine quaternisé, appelé "QPVP", qui est semble-t-il constitué d'unités répétitives formées du groupe:

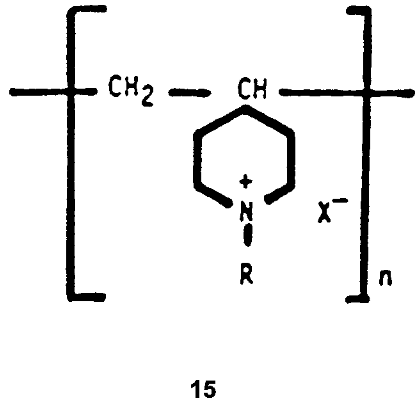

dans lequel:

n est un nombre cardinal qui est proportionnel au poids moléculaire;

R est un radical alkyle en $C_1$-$C_{20}$; et

X est un anion acceptable du point de vue cosmétique tel que halogénure, sulfate ou carboxylate,

qui peut être obtenu par quaternisation puis ensuite polymérisation de vinylpyridine d'une manière connue en soi;

(iii) polychlorure de méthacrylamidopropyltriméthylammonium, appelé "Clairquat-1" (dénomination commerciale), qui, semble-t-il, est constitué d'unités répétitives formées du groupe:

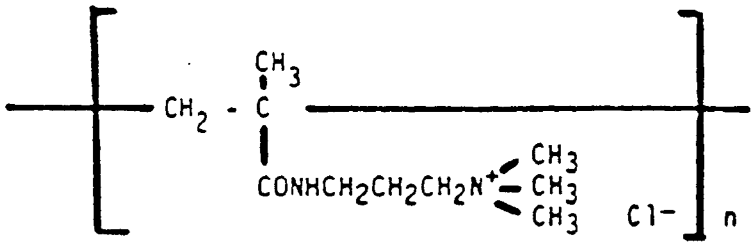

dans lequel n est un nombre cardinal qui est proportionnel au poids moléculaire;

(iv) poly(vinylamine) quaternisé, appelé "QPVAMINE", qui, semble-t-il, est constitué d'unités répétitives formées du groupe:

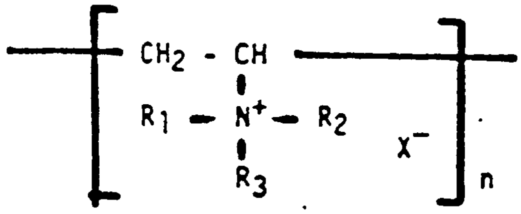

dans lequel:

$R_1$, $R_2$ et $R_3$, identiques ou différents, sont for- més de radicaux alkyles en $C_1$-$C_{20}$; et

X est un anion acceptable du point de vue cosmétique, tel que halogénure, sulfate ou carboxylate; et

(v) poly(éthylèneimine) quaternisée, appelée "QPEMINE", qui, semble-t-il, est constituée de n unités répétitives formées du groupe monomère:

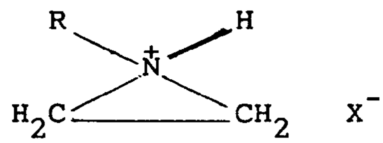

dans lequel:

n est un nombre cardinal qui est proportionnel au poids moléculaire du polymère;

R est un radical alkyle en $C_1$-$C_{20}$; et

X est un anion acceptable du point de vue cosmétique tel que halogénure, sulfate ou carboxylate;

(b) d'environ 0,5 à environ 30% d'un agent tensio-actif amphotère ou anionique hydrosoluble, ces constituants (a) et (b) formant dans un milieu aqueux dilué un produit de réaction pratiquement insoluble dans l'eau;

(c) un solvant organique en une quantité qui maintient la composition aqueuse de teinture du cheveu sous forme d'une solution préalablement à sa dilution en vue de son emploi, cette dilution étant réalisée avec une quantité d'eau qui produit la précipitation sur le cheveu du produit de réaction insoluble dans l'eau; et

(d) de l'eau en une quantité maximale insuffisante pour précipiter le produit de la réaction dans la composition de teinture du cheveu, préalablement à son emploi;

ladite composition de teinture du cheveu présentant un pH de 8 ou davantage et étant adaptée, après application au cheveu et en présence de ladite quantité d'eau de dilution, pour produire ladite précipitation sur le cheveu du produit de la réaction insoluble dans l'eau, pour teindre et également durablement conditionner le cheveu.

**2.** Une composition de teinture de cheveu selon la revendication 1, dans laquelle ledit intermédiaire de teinture du cheveu est un intermédiaire de teinture de cheveu agissant par oxydation, un colorant direct ou un mélange d'un intermédiaire colorant agissant par oxydation et un colorant direct.

**3.** Un procédé de teinture et de conditionnement durable de cheveu qui consiste à appliquer au cheveu la composition colorante selon l'une quelconque des revendications 1 à 2 et, après une période prédéterminée, à rincer le cheveu avec de l'eau pour ainsi précipiter le produit de la réaction insoluble dans l'eau et le déposer sur le cheveu.